# EUROPEAN PATENT SPECIFICATION

(11) **EP 1 392 150 B1**
(45) Date of publication and mention of the grant of the patent: **27.12.2006**
(21) Application number: 02734148.6
(22) Date of filing: 30.04.2002
(51) Int. Cl.: A61B 1/00, G01N 33/68

(54) **IMPROVEMENTS TO DIAGNOSIS OF ACUTE MYOCARDIAL INFARCTION AND OTHER CLINICAL CONDITIONS**
VERBESSERUNGEN DER DIAGNOSE DES AKUTEN MYOKARDINFARKTS UND ANDERER KLINISCHER ZUSTÄNDE
AMELIORATIONS APPORTEES AU DIAGNOSTIC DE L'INFARCTUS AIGU DU MYOCARDE ET D'AUTRES CONDITIONS CLINIQUES

(30) Priority: 04.05.2001 US 849956
(43) Date of publication of application: 03.03.2004
(73) Proprietor: Ischemia Technologies, Inc., Waltham, MA 02453 (US)
(72) Inventor: CROSBY, Peter, A., Denver, CO 80206 (US); MORRIS, Deborah, L., Boulder, CO 80303 (US)
(74) Representative: UEXKÜLL & STOLBERG
(86) International application number: PCT/US2002/013906
(87) International publication number: WO 2002/089656

(56) References cited:
- WO-A-00/20840
- US-A- 5 710 008
- US-B1- 6 235 489
- CHRISTENSON R H ET AL: "Characteristics of an albumin cobalt binding test for assessment of acute coronary syndrome patients: a multicenter study" CLINICAL CHEMISTRY, AMERICAN ASSOCIATION FOR CLINICAL CHEMISTRY. WINSTON, US, vol. 47, no. 3, March 2001 (2001-03), pages 464-470, XP002961913 ISSN: 0009-9147
- WU A H ET AL: "Analysis of the Albumin Cobalt Binding (ACB) test as an adjunct to cardiac troponin I for the early detection of acute myocardial infarction." CARDIOVASCULAR TOXICOLOGY. UNITED STATES 2001, vol. 1, no. 2, 23 August 2001 (2001-08-23), pages 147-151, XP008029899 ISSN: 1530-7905
- CIN V GOKHAN ET AL: "The prognostic value of serum troponin T in unstable angina" INTERNATIONAL JOURNAL OF CARDIOLOGY, vol. 53, no. 3, 1996, pages 237-244, XP002277249 ISSN: 0167-5273
- TAKAHASHI M. ET AL.: 'Rapid and sensitive immunoassay for the measurement of serum S100B using isoform-specific monoclonal antibody' CLINICAL CHEMISTRY vol. 45, no. 8, 1999, pages 1307 - 1311, XP002167762
- JONSSON H. ET AL.: 'S100B as a predictor of size and outcome of stroke after cardiac surgery' ANNALS OF THORACIC SURGERY vol. 71, May 2001, pages 1433 - 1437, XP002962263
- MORROW ET AL.: CLINICAL CHEM, vol. 49, no. 4, 2003, pages 537-539,

## Description

### FIELD OF THE INVENTION

The subject invention relates to the detection and diagnosis of ischemia or acute myocardial infarction in patients presenting with acute coronary syndrome, for example, to the emergency room of a hospital.

### BACKGROUND OF THE INVENTION

Each year in the United States, approximately six million people present to a hospital emergency room (ER) with Acute Coronary Syndrome (ACS). Acute Coronary Syndrome presents as a constellation of symptoms such as chest pain of suspected cardiac origin, shortness of breath, inability to maintain physical exertion, sense of dread, pain or tingling on the left arm, and may also be accompanied by clinical signs such as altered electrocardiogram. The most common presentation is chest pain of suspected cardiac origin, often referred to by its clinical description of angina pectoris. Chest pain is the number two reason for emergency room presentation, accounting for about eight percent of all patients. The chest pain patient presents a diagnostic nightmare for the emergency room physician. On one hand, if the patient really is having a heart attack, a missed diagnosis may result in poor consequences for the patient including death. On the other hand, if the patient is not having a heart attack and the physician keeps the patient in the hospital for a long time performing many diagnostic tests, the patients will consume precious health care resources that could be better spent on others. In fact, it is estimated that diagnosis of chest pain patients represents about $6 billion of wasted resources in the US alone.

The term "infarct" or "infarction" means a region of tissue which is dead and non-functional. For example, it is possible to have a brain infarct as a result of a stroke, or a bowel infarct as a result of severe bowel ischemia. A myocardial infarct (MI) is a region of dead heart muscle which is therefore unable to contribute to the pumping function of the heart. The term "heart attack" usually refers to an acute myocardial infarction or AMI, which is the emerging or developing MI.

As a person ages, there is often a buildup of fatty plaque in the coronary arteries. The plaque is usually due to deposition of cholesterol from the blood, and the risks of high cholesterol are well known in society. A plaque often consists of a soft core, with a harder membrane overlying it. At some time, a plaque may become unstable and rupture. A ruptured plaque will trigger a cascade of reactions in the blood, leading to a clot or thrombus formation. The thrombus may be carried downstream in the coronary artery circulation, which becomes progressively narrower. Eventually, the thrombus will occlude a coronary artery, disrupting circulation and preventing blood supply to the cardiac muscle or myocardium.

Ischemia is the condition of imbalance between oxygen supply and demand. Ischemia can be transitory or continuous. In the case of myocardial ischemia, the oxygen supply is provided by the blood flow in the coronary arteries. The demand may depend on the physical exertion of the person. Thus, ischemia can result from increased demand with a limited supply, or from suddenly restricted supply with constant demand, as may occur with plaque disruption and thrombus formation in a coronary artery. The first case is often referred to as stable angina. This word "stable" refers to the fact that the angina is reproducible because the restriction in supply is stable, and the ischemia can be reversed by simply ceasing the activity. If the coronary artery flow is inadequate to supply the oxygen demands of the heart during minimal activity as in the second case, the chest pain from the resulting ischemia is referred to as unstable angina. In this case, the ischemia cannot be stopped by ceasing activity, and it may deteriorate to something worse, such as acute myocardial infarction.

Once the blood supply to the myocardium is restricted, the myocardium becomes starved of oxygen, leading to ischemia. In the early stages, the tissue is reversibly ischemic, meaning that with resumption of blood supply the tissue will recover and return to normal function. After a while, the tissue becomes irreversibly ischemic, meaning that even if the blood supply is restored, the tissue is beyond salvation, and will inevitably die. Finally, the tissue dies (i.e., becomes necrosed), and forms part of the myocardial infarct. In fact, myocardial infarction is defined as "myocardial cell death due to prolonged ischemia."

Patients presenting with chest pain or ACS may in fact be having stable angina, unstable angina, or AMI. The optimal therapy for each of these patient types and the urgency for therapy is quite different, hence rapid diagnosis has enormous clinical importance.

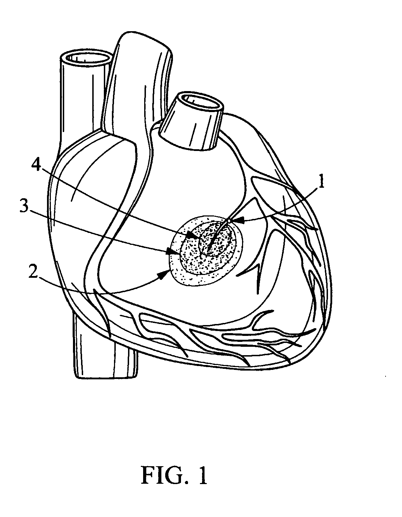

The events which occur in an AMI are illustrated diagramatically in Figure 1. An occlusion of a coronary artery (1) results in occluded flow. Tissue becomes first reversibly ischemic, then irreversibly ischemic, and finally necrosed (dead). The tissue which has been ischemic for the longest time is that which dies first. Because much of the myocardial tissue is supplied via capillaries, regions furthest from the site of occlusion are the last to receive oxygenated blood, and therefore are ischemic for shorter time than the areas closer to the site of occlusion. Thus, there are several zones of conditions proceeding in the tissue downstream from the coronary artery occlusion. The zone furthest away is reversibly ischemic (2), progressing to irreversibly ischemic (3), then finally necrosed (4). Eventually the entire region of tissue becomes necrosed with no remaining ischemic tissue, and there is a complete infarct.

Until recently, the diagnosis of an MI was done retrospectively. The criteria established by the World Health Organization (WHO) defined MI as any two of the three characteristics of (a) typical symptoms (i.e., chest discomfort), (b) enzyme rise, and (c) typical ECG pattern involving the development of Q-waves (an indication of necrosed myocardium). With these criteria, which were established some years ago, the "enzyme rise" refers to the rise of serum levels of creatine kinase (CK) or its more cardiac specific isoform CK-MB. CK-MB is a serum marker of necrosis. As a heart muscle cell dies as a result of prolonged ischemia, the cell membrane ruptures, releasing the cytosolic contents into the extracellular fluid space, then into the lymphatic system, from where it enters the bloodstream. CK-MB is one of the molecules released from dead cardiac muscle cells.

Since the WHO criteria were first promulgated, new biochemical markers of cardiac necrosis have been discovered and commercialized. (For a complete description of many of these markers, see Wu, A.H.B. (ed.) *Cardiac Markers,* Humana Press ISBN 0-89603-434-8, 1998). The most specific cardiac markers so far developed are the cardiac troponins. These are proteins which are part of the contractile apparatus of myocardial cells. Two versions, cTnI and cTnT have been commercialized, and shown to be very specific for detection of even small amounts of myocardial damage. The cardiac troponins, similar to CK-MB, are released from dead cardiac muscle cells when the cell membrane ruptures, and are eventually detectable in the blood. Necrosis can certainly occur as a result of a prolonged myocardial ischemia, but can also result from myocardial cell damage from other causes such as infection, trauma, or congestive heart failure. Thus, the observation of an increase in cardiac markers of necrosis alone does not lead to a definitive diagnosis of myocardial infarction.

The cardiac markers described above are excellent markers of necrosis, but are not markers of ischemia. However, there is much confusion in the medical community and in the literature on this point, and it is not uncommon to see references to troponin, CK-MB and myoglobin (another cardiac marker of necrosis) being described as being released as a result of ischemia. Although this is true in the sense that necrosis is always preceded by ischemia, it is not true that ischemia always leads to necrosis. Therefore these necrosis markers are not necessarily markers of ischemia. For example, the clinical condition of "stable angina" refers to chest pain as a result of physical exertion. In other words, since ischemia is an imbalance between oxygen supply and demand, if the demand is increased to the point where it exceeds the supply (for example, as a result of narrowing but not complete blockage of a coronary artery) there will be ischemia which will not necessarily lead to necrosis. If the person stops the exertion, the demand will fall to the level which can be adequately supplied by the circulation, and the ischemia dissipates. Recently, the American College of Cardiology (ACC) and the European Society of Cardiology (ESC) published a consensus document ( Alpert, J.S. et al. (2000) J. Am. Coll. Card. 36:3) with a proposed redefinition of myocardial infarction. Part of the consensus document is a new definition of acute, evolving or emerging MI. The new definition is that either one of the following criteria satisfies the diagnosis for an acute, evolving or recent MI:
(1) typical rise and gradual fall (troponin) or more rapid rise and fall (CK-MB) of biochemical markers of myocardial necrosis with at least one of the following:
   (a) ischemic symptoms;
   (b) development of pathologic Q-waves on the ECG;
   (c) ECG changes indicative of ischemia (ST segment elevation or depression); or
   (d) coronary artery intervention (e.g., coronary angioplasty); or
(2) pathologic findings of acute MI.

Implicit in this definition is the idea that an AMI includes both an ischemic component and a necrosis component. The problem is that although there are excellent biochemical markers of necrosis (i.e., troponin), there are no acceptable biochemical markers of ischemia, and therefore reliance is made on clinical impressions combined with symptoms and changes in the ECG. The fact that troponin is *not* a marker of ischemia is highlighted in the consensus document which states "these biomarkers reflect myocardial damage but do not indicate its mechanism. Thus an elevated value in the absence of clinical evidence of ischemia should prompt a search for other causes of cardiac damage, such as myocarditis."

The difficulty is that cardiac ischemia is extremely difficult to diagnose. The National Heart Lung and Blood Institute (NHLBI) of the US National Institutes of Health (NIH) created a National Heart Attack Alert Program (NHAAP) in the early 1990s. In 1997, a working group of the NHAAP published a book in which was presented an evaluation of all technologies available at the time for identifying acute cardiac ischemia in the emergency department (Selker, H.P. et al. (1997) A Report from the National Heart Attack Alert Program (NHAAP) Coordinating Committee Blackwell Science ISBN 0-632-04304-0). The key reason for this report was that new technologies for reperfusion (in particular percutaneous transluminal coronary angioplasty or PTCA, and a whole class of thrombolytic drug therapies such as TPA (tissue plasminogen activator) and streptokinase) had shown that dramatic improvements in mortality and morbidity were related to the interval between the onset of chest pain and the start of therapy. This is clearly because the earlier therapy can be applied, the more of the myocardial tissue is still reversibly ischemic instead of necrosed, and therefore there is higher likelihood that it will recover if blood supply is restored. Obviously, the key to reducing the time to therapy is to improve the performance of diagnostic tests in the emergency department (ED) such that the diagnosis can be made earlier while reversible ischemia is still present. In fact, the introduction of the NHAAP book states that "identifying only AMI would miss a large number of ED patients at significant and immediate cardiac risk."

The standard of care and the most widely accepted tool for diagnosis of cardiac ischemia in the ED is the standard twelve lead electrocardiogram (ECG or EKG). ECG suffers from imperfect sensitivity and specificity for acute cardiac ischemia, and when interpreted using stringent criteria for AMI, sensitivity drops to 50% or below. Other tools which have been investigated but not yet well accepted include variations on the ECG or algorithms involving the ECG, cardiac markers such as CK-MB and TnI, radionuclide myocardial perfusion imaging (MPI) using ⁹⁹Tc sestamibi and thallium, ECG exercise stress test, and ultrasound echocardiography. None of these has been shown to have consistently reliable sensitivity and specificity to the point where it has been accepted as standard of care. Furthermore, some technologies such as MPI, while offering relatively good accuracy, are expensive and have limited availability.

Thus, there is both an urgent clinical need and a strong economic need to find reliable biochemical markers of ischemia which can improve the diagnosis of AMI, in particular which may lead to an earlier diagnosis to allow therapeutic intervention, or to an earlier rule-out to prevent wasted health care resources.

There have been several attempts to develop a device and/or algorithms for diagnosing AMI in chest pain patients (see, for example, Jackowski, G., US Patent 5,710,008 (1998)). The '008 patent describes a method and a device for using a combination of at least three biochemical markers in conjunction with an algorithm for diagnosis of AMI. However, in the entire list of proteins that Jackowski refers to in his Table 3, not one is a true marker of ischemia, and some, if not most, are clearly markers of necrosis, not ischemia, although myocardial necrosis is the result of prolonged ischemia.

In any case, each of the markers referred to by Jackowski are molecules which are released when rupture of the cell membrane releases cytosolic contents into the extracellular fluid space, and eventually into the bloodstream. Furthermore, Jackowski requires the use of three biochemical markers with an immunoassay detection method. At least two of the three detection methods are required to recognize markers released from cardiac tissue as a result of myocardial infarction. Although Jackowski claims that his device uses an "ischemic marker", in fact the term is misleading because the markers Jackowski describes are markers of the necrotic consequences of ischemia, not the presence of ischemia. Jackowski suggests (see Claim 10) that the "cardiac specific ischemic marker is Troponin - I", which is clearly in error since Troponin I is a marker of necrosis (cell death) as a result of prolonged ischemia, and is not a marker of ischemia *per se.*

Cardiac Troponin has been accepted as the "gold standard" biochemical marker for diagnosis of acute myocardial infarction. The clinical performance of Troponin I has been reported by many publications, and by many manufacturers of troponin assays. Table 1 contains a summary of clinical performance of some troponin assays as reported in package inserts provided by the manufacturers.

Note that clinical data in most package inserts is reported in time period after symptom onset, not time period after the presentation or first draw. Reported time of symptom onset is often considered unreliable, as many patients do not remember time of onset. A small number of package inserts (e.g., the Abbott AxSym) also report the data as time periods after presentation, but this was not the norm. Calculation of sensitivity and specificity for a slow rising marker like troponin is improved by using time from presentation (since more time has elapsed for the marker to rise). Sensitivity and specificity for a fast rising marker might be degraded by using time from presentation (because the marker may have fallen in the time from symptom onset to presentation).

**Table 1**

| ***Sensitivity*** | | | | | | | | | |
|---|---|---|---|---|---|---|---|---|---|
| **Hours After Symptom Onset** | | **0-4** | | **5-11** | | **12-23** | | **>24** | |
| Manufacturer | **Cutoff ng/mL** | N | % | N | % | N | % | N | % |
| Dade Stratusⁱ | 1.5 | 72 | 37.5 | 117 | 83.8 | 177 | 92.7 | 107 | 89.7 |
| Beckman Access TnIⁱⁱ | 0.15 | 37 | 48.6 | 31 | 83.3 | 31 | 83.9 | 31 | 74.2 |
| First Medical Alpha Dxⁱⁱⁱ | 0.4 | 73 | 29 | 191 | 75 | 221 | 95 | 112 | 87 |
| | | **0-5** | | **5-12** | | **12-24** | | **24-48** | |
| Abbot AxSym^{iv} | 2.0 | 14 | 42.9 | 23 | 82.6 | 33 | 87.9 | 36 | 91.7 |

| ***Specificity*** | | | | | | | | | |
|---|---|---|---|---|---|---|---|---|---|
| **Hours After Symptom Onset** | | **0-4** | | **5-11** | | **12-23** | | **>24** | |
| Manufacturer | **Cutoff ng/mL** | N | % | N | % | N | % | N | % |
| Dade Stratusⁱ | 1.5 | 75 | 100 | 158 | 99.4 | 269 | 98.9 | 73 | 97.3 |
| Beckman Access TnIⁱⁱ | 0.15 | 215 | 97.2 | 196 | 94.9 | 115 | 93.0 | 20 | 93.1 |
| First Medical Alpha Dxⁱⁱⁱ | 0.4 | 73 | 93 | 191 | 94 | 221 | 95 | 112 | 99 |
| | | **0-5** | | **5-12** | | **12-24** | | **24-48** | |
| Abbot AxSym^{iv} | 2.0 | 52 | 92.3 | 81 96.3 | | 134 | 94.8 | 74 | 95.9 |

| ***Sensitivity*** | | | | | | | | | |
|---|---|---|---|---|---|---|---|---|---|
| **Hours After Presentation** | | **0-5** | | **5-12** | | **12-24** | | **>24** | |
| Manufacturer | **Cutoff ng/mL** | N | % | N | % | N | % | N | % |
| Abbot AxSym^{v} | 2.0 | 113 | 64.6 | 77 | 92.2 | 112 | 89.3 | 90 | 93.3 |
| ***Specificity*** | | | | | | | | | |

| **Hours After Presentation** | | **0-5** | | **5-12** | | **12-24** | | **>24** | |
|---|---|---|---|---|---|---|---|---|---|
| Manufacturer | **Cutoff ng/mL** | N | % | N | % | N | % | N | % |
| Abbot AxSym^{v} | 2.0 | 443 | 94.6 | 372 | 93.8 | 368 | 94.0 | 179 | 94.4 |

| | | | | | | | | | |
|---|---|---|---|---|---|---|---|---|---|
| ⁱ Dade Stratus Package insert, no document number or date identified ⁱⁱ Beckman Access Troponin 133320 Package Insert Document # 1051468 1997 ⁱⁱⁱ First Medical Alpha Dx Cardiac Panel Test Kit Package Insert doc # 88-0001 ^{iv} Abbott AxSym Troponin 1 List No. 3C29, Doc # 69-0176/R1 December 1997 ^{v} Abbott AxSym Troponin I List No. 3C29, Doc # 69-3119/R3 December 1997 | | | | | | | | | |

Clearly, although troponin is an exquisitely sensitive marker for cardiac necrosis, its clinical utility, especially in the early period following onset of chest pain (i.e., immediately after the coronary artery occlusion leading to ischemia) is limited by the slow kinetics of the marker itself, and the fact that it is a marker for necrosis, not ischemia, and therefore released late in the clinical sequence.

Attempts to obtain better diagnosis of AMI using combinations of results from these biochemical markers of necrosis have been described. For example, Shah et al., US Patent No. 5,382,515 (1995), describe an algorithm using sequential closely spaced measurements of different isoforms of creatine kinase to determine both the presence and the time of an AMI. The concept was expanded by Groth, T. et al., US Patent No. 5,690,103 (1997), who describe the use of an algorithm implemented by a neural network whose inputs are several closely spaced measurements of several markers released from necrotic tissue (CK-MB and troponin). Although this method may be beneficial in that it is still better than measurement of a single necrosis marker, or multiple necrosis markers at a single time, it is still not possible to make the determination until at least three hours have passed, and does not work for detection of ischemia since only necrosis markers are used.

A similar approach (although without a neural network) was proposed by Armstrong et al. (US Patent No. 6,099,469 (2000)), although in this case the algorithm is designed to run on the computer embedded in an automated laboratory analyzer, and suggests which test should be performed next. Again, the Armstrong invention suffers from the limitation that it uses only markers of necrosis, and requires multiple sequential measurements to achieve adequate performance.

Ohman et Al. (US Patent No. 6,033,364 (2000)) described algorithms using of existing markers of necrosis which have also been used to assess reperfusion after thrombolytic therapy. In this invention, an algorithm using sequential measurements of a necrosis marker (CK-MB) and a model based on the rise and fall kinetics of CK-MB can determine when therapy has allowed restoration of coronary artery flow and therefore arrested the growth of infarcted tissue and hence release of further markers of necrosis.

Christenson et al., Clinical Chemistry 47:3, 464-470, 2001, describe the use of an albumin cobalt binding (ACB) test for assessment of acute coronary syndrome patients. The ACB test is said to be an early indicator of myocardial ischemia before necrosis. The results of the albumin cobalt binding test were correlated to the results of a cardiac troponin I (cTnI) test. It is suggested that an early ACB test can be predictive of troponin tests taken at 6-24 hours.

### SUMMARY OF THE INVENTION

It is an objective of the present invention to use a true biochemical marker of ischemia in conjunction with one or more biochemical markers of necrosis to allow an earlier and reliable diagnosis of ischemia and acute, emerging, or developing myocardial infarct.

The subject invention comprises a method for diagnosing a clinical event occurring in a patient's tissue selected from ischemia or myocardial infarction by obtaining from the patient at least one sample of a substance stream (such as the bloodstream), wherein the stream is in contact with the tissue in the patient; conducting at least two *in vitro* diagnostic tests on the sample, one of which is an assay for a molecule released from the tissue during the clinical event, and one which is an assay for a molecule that is present in the stream and is modified by the clinical event; and combining the results of the foregoing assays using an algorithm to provide; for example, a positive or negative diagnosis of the clinical event. An "algorithm" as used herein refers to the steps involved in making a diagnosis of the occurrence of a clinical event.

Depending on the nature of the clinical event and the sensitivity and specificity of the two assays for their target molecules, the algorithm provides a diagnosis of ischemia or myocardial infarction, said algorithm comprising:
diagnosing ischemia with either early infarct of less than 4 - 6 hours or stable angina, if the modified albumin result is positive and the necrosis marker result is negative; and
diagnosing myocardial infarction if the ischemia modified albumin result is positive or negative and at least one of the necrosis marker results is positive.

The substance stream refers to any flowing body tissue or fluid including but not limited to urine, saliva, tears, semen, mucus, feces, blood, lymph, serum, plasma and expired breath.

The clinical event is an acute myocardial infarction (AMI) or ischemia. The assay for a molecule released from the tissue during the clinical event can be an assay for a necrosis marker selected from the group including but not limited to troponin, CK-MB and myoglobin, and the assay for a molecule that is present in the stream and which is modified by the clinical event, is an assay for ischemia modified albumin (which is referred to herein as IMA™). The patient sample can be blood, serum or plasma. The assay for ischemia modified albumin can be, for example, the Albumin Cobalt Binding (ACB) test or an immunoassay specific for ischemia modified albumin, i.e., using antibodies directed to the altered N-terminus of albumin.

Diagnosis of acute myocardial infarction may be ruled out by obtaining a sample of a patient's blood, serum or plasma, conducting at least one *in vitro* assay for an ischemic marker and at least one *in vitro* assay for a necrosis marker, and combining the results of the assays using an algorithm to provide a negative diagnosis of acute myocardial infarction. A negative diagnosis may be made where all ischemia marker tests and all necrosis marker tests are negative, or where the majority of both the ischemic marker tests and necrosis marker tests are negative. As is discussed herein, this can have the advantage of a high negative predictive value (NPV), making it useful in ruling-out the occurrence of an AMI. Ruling-out an AMI relatively early after patient presentation at an emergency room can lead to early patient release and conservation of medical resources.

Further in relation to myocardial infarction the outcome of a troponin assay in a patient presenting with chest pain may be predicted, comprising obtaining a patient blood, serum or plasma sample, conducting an ACB Test on the sample to measure ischemia modified albumin, and predicting the outcome of the troponin assay on a patient blood, serum or plasma sample taken within the next 2-24 hours, wherein the prediction is positive if the ACB Test result is above an ACB test decision point, and wherein the prediction is negative if the ACB Test result is below the ACB test decision point. As is described herein, the ACB Test decision point is determined from sensitivity and specificity data obtained from studies of patients presenting with symptoms characteristic of a myocardial ischemia.

Diagnostic devices that are useful in the foregoing methods comprise a first and second flow path, wherein each flow path comprises an application zone on a carrier media for application of the sample, and a test zone in fluid communication with the application zone. The test zone of each flow path contains non-diffusively bound reagents necessary for performing an assay for the presence of either the released molecule or the modified molecule. Non-diffusively bound means that the reagents are immobilized or stably retained in the zone under conditions of use: Reagents can be immobilized using suitable techniques known in the art. The test zone reagents of the first flow path can detect or measure a molecule released from the patient's tissue into the stream as a result of the clinical event, and the test zone reagents of the second flow path can detect or measure a molecule that is modified by the clinical event. That is, a reagent in the test zone of the first flow path forms a binding pair with the released molecule, while a reagent in the test zone of the second flow path forms a binding pair with the modified molecule. Reagents can therefore include, for example, antigens, antibodies, receptors, peptides, proteins, ligands, single-stranded and double-stranded DNA, oligonucleotides, cDNA, mRNA, RNA and the like. The specific binding pair may not itself be detectable by visual or machine-assisted readout, but may be made so by techniques known to those skilled in the art. For example, the detectable indication can be created by an enzyme-linked assay, fluorophores, chromophores, radioisotopes, dyes, colloidal gold, colloidal carbon, latex particles or chemiluminscent agents. The detection of the binding pair may occur simultaneously with the test, or may occur in one or more subsequent steps.

The device can be a strip test and the carrier media can be a solid substrate in an elongated rectangular form. The application zone can be located at a first end of the elongated form and the test zone can be located at a second end of the elongated form. The device may further comprise a quality control zone in fluid communication with the application zone which comprises an indicator reagent for confirming the completion of the assay. In such case, the test zone may be located between the application zone and the quality control zone on the elongated form.

The device is useful in diagnosing acute myocardial infarction using a patient sample of blood, serum or plasma. As described above, the device comprises a first and second flow path, each with an application zone and a test zone in fluid communication with the application zone. The test zone reagents of said first flow path can detect or measure an ischemic marker (e.g., ischemia modified albumin) in the sample, and the test zone reagents of said second flow path can detect or measure a necrotic marker (e.g., troponin, CK-MB or myoglobin) in the sample.

The first and second flow paths may be combined, i.e., occupy the same space on the carrier media, provided that the test zones do not overlap or obscure reading of the assay results. In this embodiment, the arrangement of zones along the elongated rectangle may be the application zone, test zone for the ischemic marker or necrotic marker, test zone for the necrotic or ischemic marker (respectively), followed by the quality control zone. As is appreciated by persons of skill in the art, multiple variations in zone arrangements are possible.

### BRIEF DESCRIPTION OF THE DRAWINGS

Figure 1 is a diagrammatic illustration of the zones of reversibly ischemic, irreversibly ischemic, and necrotic tissue a short time after a coronary artery occlusion.
Figure 2 is a graph showing the time course of rise and fall of a test for ischemia modified albumin during brief cardiac ischemia induced as a result of PTCA.
Figure 3 is a graph of Sensitivity against time for the ACB Test, Troponin-I, and the two tests used in combination.
Figure 4 is a diagrammatic illustration showing the sequence.of rise and fall of ischemia modified albumin and troponin during an AMI after a coronary artery occlusion.
Figure 5A is a plot of the distribution of ACB Test results at patient presentation for the troponin-positive and -negative groups at 6-24 hours after presentation (Example 2).
Figure 5B is a Receiver Operating Characteristics (ROC) curve for the ACB Test results at patient presentation compared to the troponin results at 6-24 hours (Example 2). The ROC curve can be used to determine the optimal operating point (cutoff) for best sensitivity, best specificity, or some other combination.

### DETAILED DESCRIPTION OF THE INVENTION

Human serum albumin has been found to be modified by exposure to ischemic tissue in such a way that it is less capable of binding certain metals, in particular cobalt. The detection of such ischemia modified albumin (IMA™) is embodied in the Albumin Cobalt Binding Test (ACB™ Test) developed by Ischemia Technologies, Inc., Denver, CO. The measurement of modified metal binding ability of serum proteins (including albumin) for detection of ischemia was first described in Bar-Or, D. et al. (1993) US Patent 5,227,307, *Test for the Rapid Evaluation of Ischemic State,* and Bar-Or, D. et al. (1994) US Patent 5,290,519, *Test for the Rapid Evaluation of Ischemic States and Kit.* Further developments relating to diagnosis of ischemia have been described in US Patent applications 09/165,581, 09/165,926 and 09/165,961, each filed October 2, 1998, and PCT/US99/22905 and PCT/US99/22746, each filed October 1, 1999, and all of which are incorporated herein in their entireties by reference. Preliminary results of experiments to confirm the mechanism of IMA have also been published (Bar-Or et al. (2001) Eur. J. Biochem. 268:42-47).

There is a fundamental difference between conventional markers of necrosis such as troponin, myoglobin and CK-MB (for example, as described by Jackowski, et al., *supra)* and the use of ischemia modified albumin. In the former case, biochemical markers of necrosis are molecules available in the bloodstream some time after the cytosolic contents of a cell are released as a result of rupture of the cell membrane from necrosis. The molecules are released first into the extracellular space, from there to the lymphatic system, and thence drained into the bloodstream. In the case of IMA, albumin is circulating in blood, and is rapidly modified as a result of exposure to ischemic tissue. Therefore, there is no requirement for the cell membranes to rupture (necrosis), nor is there a long time delay between the event leading to ischemia and the time the biochemical marker can be detected in the bloodstream.

The ACB Test has been demonstrated to detect the rapid rise in IMA following a transient ischemic event, caused by percutaneous transluminal coronary angioplasty (PTCA) (Bar-Or et al. (2001) Am. H. J., *in press).* PTCA is a procedure during which a catheter is threaded into a coronary artery via radiographic guidance to the location of a coronary artery occlusion. The catheter has a long thin balloon at its tip. When in position, the balloon is inflated, pushing the plaque up against the wall of the artery, thereby increasing the size of the lumen, and restoring flow upon balloon deflation. The PTCA procedure is well accepted in clinical practice.

At the time of balloon inflation (typically 30 seconds to two or three minutes), there is no coronary artery flow. The absence of flow therefore induces temporary ischemia downstream from the site of balloon inflation. However, this short duration of ischemia does not induce the changes seen as a result of long duration ischemia, such as cell necrosis.

Bar-Or et al. (2001), *in press, supra,* investigated the response of IMA to PTCA by analyzing blood from a number of patients presenting for the PTCA procedure. Blood was taken immediately before balloon inflation (0 hours), immediately after balloon inflation (0+ hours), and then six and twenty four hours later, and a manual version of the ACB Test was performed on the samples. A group of control patients who were subjected to coronary angiography without balloon inflation (i.e., no ischemia induced) was also studied.

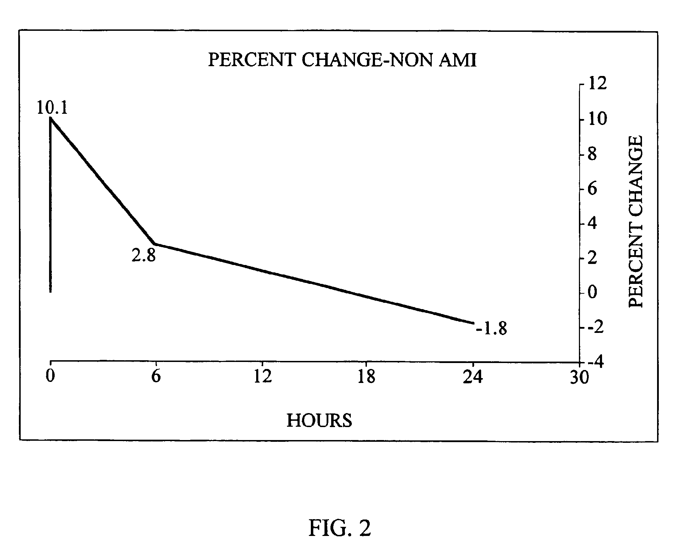

The results from 41 patients (who did not have an AMI shortly before or after the procedure) are shown in the graph in Figure 2, which plots the percent change in the results from baseline. Between before inflation and after inflation, the difference in free serum Co is highly statistically significant (p=.0001); between baseline and six hours, there is moderate significance for the difference (p=0.02); and there is no statistical difference between baseline and 24 hours later. Furthermore, troponin levels were examined from these patients, and there was no elevation of troponin above the AMI cutoff during the time frame of the experiment. The control group showed no significant difference between the test result before and after angiography without balloon inflation.

These results show that the IMA rises extremely rapidly with the onset of even a small amount of short duration ischemia, and falls back to baseline level at some time after six hours. This is consistent with our understanding that the IMA marker is *not* a marker of necrosis, nor is it a molecule released from inside the cell, rather it is a circulating molecule which is modified by exposure to ischemic tissue. For a detailed discussion of the specific changes to the N-terminus of albumin due to exposure to ischemic tissue, see PCT/US99/22905Additional studies have illustrated that the ACB Test is a strong diagnostic tool for the detection of ischemia. In a study conducted by the subject inventors and others, a group of patients presenting to the hospital emergency room with chest pain of suspected cardiac origin, or suffering from stable angina and referred for stress testing, were evaluated. This group of patients was selected such that their risk of having suffered an AMI near presentation time was minimal, and their biochemical markers of necrosis (i.e., troponin) measured from the first blood draw upon presentation were negative. In other words, there was no definitive indication that the patient was suffering from AMI.

As part of the normal diagnostic workup, the patients were subjected to myocardial perfusion imaging (MPI). With this test, the patient is injected with a solution of a molecule which has been tagged radioactively. The molecule is chosen such that it is preferentially taken up by cardiac tissue which is in normal metabolism, for example sestamibi which has been tagged with Tc⁹⁹. The patient is then imaged using nuclear medicine techniques (gamma camera, PET scan, SPECT scan and the like). The cardiac muscle which is normally perfused and undergoing normal aerobic metabolism appears bright on the image, whereas the cardiac tissue which is under-perfused (i.e., ischemic) appears dark on the image.

This technology is considered by many to be the "gold standard" for diagnosis of cardiac ischemia, however it is far from perfect, and it is known that its clinical sensitivity is on the order of 85%, and clinical specificity is approximately 75%, and the technology suffers from many limitations such as the problem of distinguishing between old infarct and new ischemic regions, both of which appear dark on the image. Furthermore, MPI is not available at all hospitals, and in particular is only rarely available on call for patients presenting to the emergency room with chest pain, due to costs and logistical difficulties. Finally, MPI is believed to have a threshold of a minimum of 10gm of affected myocardial tissue before the effect can be seen on the images, and it is possible that small areas of ischemic tissue can be missed.

The diagnostic performance of MPI can be improved if it is used in conjunction with ECG analysis. In particular, a conventional 12 lead ECG recording is considered to be diagnostic of cardiac ischemia (as opposed to necrosis) if there is at least 1mm elevation or depression of the ST segment in two leads. Finally, MPI can be performed either on patients at rest who are experiencing chest pain at the time of radioactive tracer injection, or can be performed on patients who are exercising, when the radioactive tracer is injected at the time of peak exercise (highest probability of myocardial ischemia).

In this clinical study, 50 patients were examined, and the results are shown in Table 2.

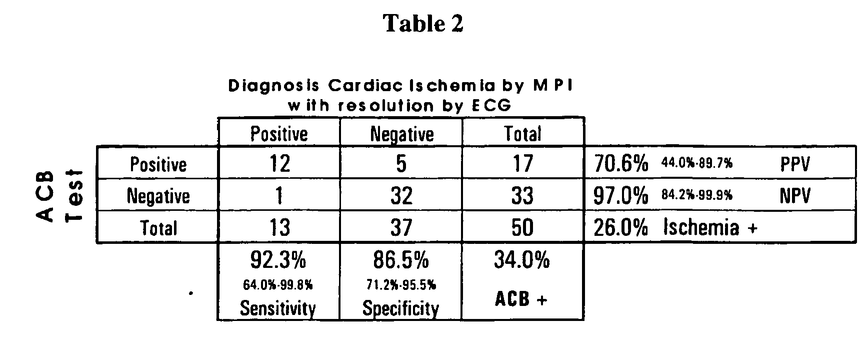

A positive diagnosis of ischemia via MPI/ECG was defined as when the MPI was positive for ischemia, or when the MPI was negative for ischemia and the ECG was positive for ischemia (ST segment changes in two leads). The sensitivity of the ACB Test to detect cardiac ischemia by this definition was calculated to be 92.3%, and the specificity was 86.5%. This indicates that the ACB Test is a strong diagnostic tool for the detection of ischemia, even without the simultaneous detection of markers of necrosis.

A further study showing the utility of the ACB Test as a diagnostic tool for ischemia is described in Bar-Or et al. (2000) J. Emerg. Med. 19:311.

As discussed above, the subject invention is directed broadly to a method of detecting a clinical event that has affected a tissue by collecting a patient sample from a substance stream that is in contact with the affected tissue, and conducting an assay for a molecule that has been released from the tissue and an assay for a molecule usually present in the substance stream which has been modified by the clinical event. The two assay results are then combined using an algorithm to diagnose the occurrence of the clinical event. It is believed that this is the first time released molecule assay results and modified molecule assay results have been combined in an algorithm to diagnose a clinical event. As applied to AMI, the patient sample is blood, serum or plasma, the released molecule is a necrotic marker (e.g., troponin, CK-MB or myoglobin) and the modified molecule is an ischemic marker (e.g., IMA). It is believed that the subject invention represents the first time the results of a true ischemia marker assay and necrosis marker assay have been combined in an algorithm to diagnose the occurrence of AMI with substantially improved sensitivity and specificity. Moreover, it is believed that it is the first time that a particular ischemic marker test, the ACB Test, has been demonstrated to have a high sensitivity and NPV in predicting the outcome of a necrosis marker (troponin) assay. These discoveries reveal the value of ischemic markers in predicting the outcome of necrotic assay markers in situations where clinical symptoms are equivocal, and the value of ischemic marker assays used in combination with necrosis marker assays in the diagnosis of AMI.

As described in detail in the Examples, it has been found that the ACB Test results for samples collected at the time of presentation of chest pain patients in an emergency room can be an early predictor of necrosis marker assay results within the next several (2-24) hours. For example, it has been shown that the ACB Test reliably predicts whether cTnI results will be positive or negative at 6-24 hours later. Early prediction is significant, particularly given the knowledge that troponin status can be utilized for reliable risk stratification (Newby et al. (1998) Circulation 98:1853; Morrow et al. (2000) Clin. Chem. 46:453), and may be useful for therapeutic guidance with inhibitors of the platelet glycoprotein IIb/IIIa receptor (Hamm et al. (1999) N. Eng. J. Med. 340:1623; Heeschen et al. (1999) Lancet 354:1757) or with low-molecular weight heparin (Lindahl et al. (1997) J. Am. Coll. Cardiol. 29:43).

In Example 1, blood samples drawn from 224 patients with chest pain at presentation at emergency rooms were tested for IMA using the ACB Test and for troponin. The patients were also tested at 6-24 hours later for troponin. All participating patients had no detectable troponin at presentation. Using an ACB Test cutoff of ≥75 units/mL, it was found that the ACB Test had a sensitivity of 83%, a specificity of 69%, a positive predictive value (PPV) of 33%, and a negative predictive value (NPV) of 96% for a later outcome of a troponin assay.

The high NPV of the ACB Test for later troponin results could allow clinicians to more safely and cost-effectively identify low-risk patients at the time of presentation at emergency rooms. In this way, the ACB Test can have a large impact for the estimated 8 million patients who present annually with symptoms suspicious for AMI (Storrow et al. (2000) Ann. Emerg. Med. 35:449). The ACB Test could bring a new dimension to the care of acute coronary syndrome patients by adding substantially to troponin measurements, which have low diagnostic sensitivity (30-50%) in the first hours after presentation (Mair et al. (1995) Clin. Chem. 41:1266; Antman et al. (1995) JAMA 273:1279). This early benefit of the ACB Test is important because the REACT study showed that the median time from onset of symptoms to presentation was only 2.0 hours for acute coronary syndrome patients, with only 25% of patients delaying presentation longer than 5.2 hours (Goff et al. (1999) Am. Heart J. 138:1046). The high sensitivity and NPV demonstrated by the ACB Test results for samples collected at presentation at the emergency room may substantially reduce delays in patient disposition from the 6-24 hours required for reliable troponin-negative classification. The ACB Test has a significant role in greatly reducing the inappropriate admission of low-risk patients.

As discussed above, another embodiment of the subject invention includes an improved method for diagnosis of AMI by conducting at least two *in vitro* tests, one of which is for ischemia, and the other of which is for necrosis, and combining the results of the tests using an algorithm to diagnose whether an AMI has occurred or is in process. Preferably, the test for ischemia is the ACB Test, and the necrosis test is a troponin assay. Alternatively, the necrosis markers can be CK-MB or myoglobin or other necrotic markers known in the art, such as those described in Wu, A.H.B. (1998), *supra.*

The algorithm provides a diagnosis of ischemia or myocardial infarction, said algorithm comprising:
diagnosing ischemia with either early infarct of less than 4 - 6 hours or stable angina, if the modified albumin result is positive and the necrosis marker result is negative; and
diagnosing myocardial infarction if the ischemia modified albumin result is positive or negative and at least one of the necrosis marker results is positive.

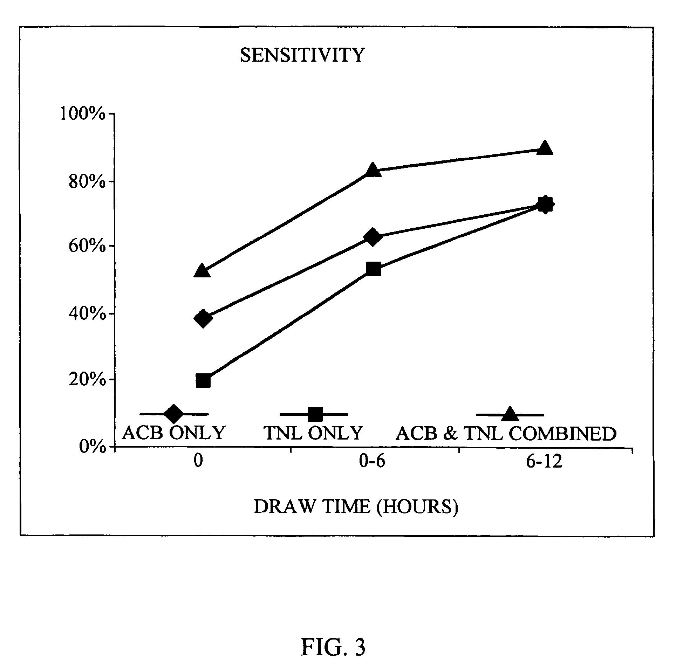

As is described in detail in Example 2, clinical trial data was used to determine the sensitivity and specificity associated with using both the ACB Test and a cardiac troponin assay in the diagnosis of an AMI. It was found that the combination of the ACB Test and troponin assay always produced a higher sensitivity and specificity in diagnosing AMI than either test alone (see Fig. 3). The ACB Test has higher sensitivity than troponin at the earlier draws, as might be expected since the ACB Test, being a test for ischemia, is a test for the earlier stages of an AMI.

Fig. 3 illustrates a surprising aspect of the subject method: the set of patients that test positive for troponin and the set of patients that test positive for IMA are not identical. The necrosis marker assay identifies AMI patients that are not identified by the ischemia marker assay, and the ischemic marker assay identifies patients that not identified by the necrosis marker assay. By employing marker assays that complement each other, the subject method enjoys advantages not employed by either assay alone. It could not have been predicted that together, the necrosis marker assay and ischemia marker assay could have improved specificity and sensitivity in the diagnosis of AMI.

Furthermore, it can be seen from the data of Example 2 that the negative predictive value (NPV) is higher when both troponin and the ACB Test are used in combination, than when either is used alone. The negative predictive value is of most interest in a test used for rule-out. For example, an NPV of 80% means that if the test is negative, there is an 80% likelihood that the patient does not have a positive diagnosis. The important thing to note in the data of Example 2 is that the combination of the ACB Test and troponin yields higher sensitivity (i.e., more patients correctly diagnosed *with* AMI) without sacrificing NPV (i.e., no loss of accuracy in diagnosis of patients *without* AMI).

The clinical study described in Example 2 was performed using a clinical chemistry assay (the ACB Test) and an immunoassay (troponin). It is possible for IMA to be detected using an immunoassay (see co-pending patent application of Bar-Or et al. PCT/US99/22905). In that case, both the IMA and the troponin immunoassay tests could be performed using the same immunoassay instrument.

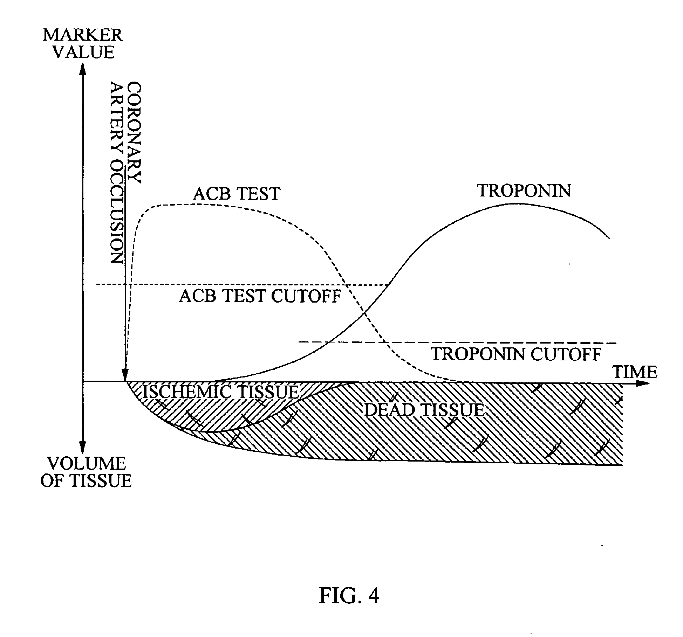

The relationship between the ACB Test and troponin during an AMI is illustrated diagrammatically in Figure 4. The bottom section of the graph represents the volume of tissue which is ischemic or necrosed. The top section of the graph represents the values of the two markers. At the time of a coronary artery occlusion (shown as the vertical arrow to the left of the horizontal time axis), some tissue immediately becomes reversibly ischemic. With the passage of a small amount of time, the tissue which has been reversibly ischemic the longest starts to become irreversibly ischemic and will eventually die. As more time passes, more and more of the tissue becomes ischemic, and more of the ischemic tissue becomes necrotic. Eventually, the volume of ischemic tissue starts to decrease, as the ischemic tissue is converted to necrotic tissue. Eventually, all the tissue affected by the coronary artery occlusion is necrotic, and there is a full blown infarct, with no ischemic tissue remaining.

A short time after the coronary artery occlusion, the ACB Test value rises above the cutoff (diagnosis level), indicating rapidly the presence of ischemic tissue. As time goes on, the ACB Test remains elevated while there is still ischemic tissue, and once all the affected tissue has converted from ischemia to necrosis, the ACB Test starts to fall. As soon as some of the ischemic tissue becomes necrotic, troponin is released and makes its way into the bloodstream. Once there is a sufficient volume of necrotic tissue, and a sufficient time has passed, the serum troponin level rises above the cutoff level.

Thus it can be seen that the combination of the ACB Test and troponin (or another marker of necrosis) yields more information than either test on its own, as illustrated in Table 3, for interpretation of results of patients with ACS.

**Table 3**

| ACB Test | Troponin | Likely Clinical Situation |
|---|---|---|
| - | - | No ischemia, no infarct |
| - | + | Late infarct - several hours or days after coronary artery occlusion |
| + | - | Ischemia. Either early infarct (<4-6 hours), or if these test results are present for a long time, angina which will not lead to infarct |
| + | + | Early infarct (>4-6 hours) |
| | | Or |
| | | Second ischemic event (eg: re-occlusion after infarct) if troponin has been elevated for some time) |

The algorithm set forth in Table 3 has the advantage over previously described algorithms for assessing AMI based on markers of necrosis in that it may be performed on measurements of only two biochemical markers taken at a single draw time instead of multiple measurements taken from sequential draws. Furthermore, since it uses a rapidly rising marker of ischemia, it is able to more rapidly assess patients who are ischemic but who have not yet proceeded to necrosis, and in fact is able to provide a confirming diagnosis of ischemia in chest pain patients without AMI which is not possible with markers of necrosis, especially since necrosis markers are not elevated in patients with stable angina, and are often not elevated or only slightly elevated in patients with unstable angina.

In addition, the algorithm set forth in Table 3 yields more information than just the presence or absence of a single clinical condition. The combination of these two markers, combined with a knowledge of the kinetics, allows deduction of information such as when in the time course of a clinical episode the samples were obtained.

Finally, it overcomes one of the major limitations of the use of troponin, and that is that because troponin falls slowly after infarct (usually over several days), it is extremely difficult to diagnose re-infarct or subsequent ischemic events. As an example, consider a patient who present with chest pain, is diagnosed as having an AMI and is treated with some sort of reperfusion therapy (e.g., thrombolytics, PTCA, stent or surgery). If this patient presents with another episode of chest pain two or three days after the initial event, with present biochemical markers it is extremely difficult to determine if this is another event, because troponin (and possibly CK-MB) will still be elevated due to cardiac damage from the initial AMI. However, since the IMA marker falls so rapidly after an episode, a patient's discharge value of the IMA marker is likely to be within normal range. Thus, if the patient presents with another episode, if the IMA marker is elevated upon presentation the second time, then it is likely to be a second event.

Since the algorithm described above requires evaluation of whether the tests results are above or below a predetermined cutoff, it is not strictly necessary for the test method to produce a quantitative result, although of course a quantitative result gives additional information about timing of the event, particularly if sequential measurements are taken.

The preferred embodiment described above is for more rapid and accurate diagnosis of AMI using a circulating protein biochemical marker of ischemia and a protein released from necrosed cells (i.e., troponin). Other biochemical markers of necrosis can be used, such as CK-MB or myoglobin, or any of the markers identified in Wu, A.H.B. (1998), *supra.* It may also be possible that other molecules circulating in blood might be discovered which are modified as a result of an ischemic event, in which case that biochemical marker could be used instead of or in addition to IMA.

In a further embodiment, results from measurements of additional biochemical markers can be utilized to provide extra information over and above the presence or absence of a clinical event. Table 4 shows how the addition of the results of an assay for the IMA marker can help resolve different clinical conditions which are confusing when using troponin (TnI) and myoglobin (Myo) alone in diagnosis of patients presenting to the emergency room with chest pain of suspected cardiac origin.

**Table 4**

| **TnI** | **Myo** | **IMA** | **Probable Diagnosis** |
|---|---|---|---|
| - | - | - | Non-cardiac or non-ischemic cardiac event |
| | | + | Ischemic event |
| - | + | - | Non ischemic muscle injury |
| | | + | Ischemic event with muscle injury or early infarct |
| + | - | - | Late infarct or non-ischemic cardiac damage |
| | | + | Ischemic event, following earlier infarct |
| + | + | - | Post infarct without ischemia |
| | | + | Post infarct with ischemia |

In a further embodiment, serial determinations of an assay for an IMA marker in conjunction with serial determinations of an assay for troponin can yield information about the time course of a cardiac ischemic event. Reference to Figure 4 shows diagramatically the rise and fall of the IMA marker and troponin for a single event of coronary artery occlusion. This algorithm is described in Table 5, along with possible therapies based on the time from occlusion. Notice that the addition of the IMA marker can help to choose between therapies where the choice is not available using TnI alone.

**Table 5**

| **TnI** | **IMA** | **Probable Diagnosis** | **Possible Therapy** |
|---|---|---|---|
| Below Cutoff | Above cutoff, and rising | Very early stage of ischemic event | Thrombolytic therapy |
| Below cutoff | Above cutoff and steady state | During Ischemic event | Thrombolytic or revascularization therapy |
| Rising | Steady state | During ischemic event, >2 hours from start of event | Revascularization therapy |
| Rising | Falling | No reversible ischemia remaining, tissue is infarcted | Maybe revascularization |
| Rising | Below Cutoff | Late stages of AMI, >2 hours, probably < 12 hours | Nothing |
| Failing | Below Cutoff | Late stage of AMI, probably >12 hours | Nothing |
| Falling | Rising | Second ischemic event | Thrombolytic therapy |

Although the method has been described in terms of using circulating and released biochemical markers which are detected in blood (with serum or plasma), the invention is not restricted to this type of sample. Other substance streams (body fluids or tissue samples)

Devices useful in the foregoing methods comprise a first and second flow path. Each flow path has an application zone on a carrier media for application of the sample, and a test zone in fluid communication with the application zone. The application zone of the first and second flow paths may be at the same situs. The test zone of each flow path contains reagents necessary for performing an assay for the presence of a molecule. The test zone reagent of the first flow path detects or measures a molecule released from the tissue undergoing the clinical event into the stream, and the test zone reagent of the second flow path detects or measures a molecule already present in the stream that is modified by the clinical event. The test zone reagents in the first and second flow path bind to or react with the released molecule in such a manner as to manifest a detectable or measurable change using methods known in the art, e.g., colorimetric methods, immunoassays including ELISA, enzyme-cofactor (avidin and biotin) methods and the like. The test zone can provide one or more reagents as appropriate to accomplish the indicator function. The test zone can be read by the human eye or by other methods known in the art.

The first and second flow paths may occupy the same space on the device, provided that the first and second test zones are spatially distinct and separately readable.

The device may be a strip test and the carrier media can be a solid substrate in an elongated rectangular form. The solid substrate can be any suitable material known in the art, including paper, nitrocellulose or other porous inert material. In one embodiment, the device has the application zone located at a first end of the elongated form and the test zone located at a second end of the elongated form. The device can also have a quality control zone in fluid communication with the application zone. The quality control zone has an indicator reagent for confirming the completion of the assay. In such case, the device could have the test zone located between the application zone and the quality control zone on the elongated form. The quality control zone may be a single zone that is common to the first and second flow paths.

The indicator reagent used in the quality control zone is typically a material that is sensitive to the presence of the sample. It is generally a material that will change color in response to the presence of some moiety in the sample solution. Examples of such a reagent include, but are not limited to, pH indicator dyes, dyes sensitive to the presence of proteins, and dyes sensitive to hydration states. A successful test run will result in a detectable indication within the quality control zone, also called a quality control confirmation.

The device may be useful in diagnosing acute myocardial infarction using a patient sample of blood, serum or plasma. In this case, the device comprises a first and second flow path, wherein each flow path comprises an application zone on a carrier media for application of the sample, and a test zone in fluid communication with the application zone, the test zone of each flow path providing reagents necessary for performing an assay for the presence of a molecule. The test zone reagents of the first flow path detect or measure the ischemic marker in the sample, and the test zone reagents of the second flow path detect or measure a necrotic marker in the sample. If the ischemic marker is ischemia modified albumin, the assay can be the ACB Test or an immunoassay specific for ischemia modified albumin. The necrosis marker can be any cardiac necrosis marker known in the art, including troponin, CK-MB and myoglobin.

An example of a "strip test" dry chemistry format is described by Jackowski, G., US Patent No. 5,290,678. The strip test described therein could be configured such that the visible indication of the immunoassay result appeared if the concentration of the marker being measured is above the cutoff. In that case, if the strip test were configured with an immunoassay for the IMA and an immunoassay for troponin, then the presence of either of the visible indications would mean a diagnosis of "probably AMI", and the absence of any visible indications would mean "probably not AMI". In this way, two of the key limitations of Jackowski's ('008) original invention would be overcome: (1) there is only the requirement for two markers, and (b) the time between the onset of chest pain and the time at which a reliable diagnosis could be performed would be shorter.

### EXAMPLES

### Example 1 - The ACB Test in the Prediction of Troponin Levels

A study was conducted to examine the ability of the ACB Test result for samples collected at presentation to predict a cardiac troponin-positive result in the subsequent 6-24 hours timeframe, which reflects myocardial injury caused by ischemia, or a cardiac troponin-negative outcome 6-12 hours after presentation. The study results described herein are published in Christenson et al. (2001) Clin. Chem. 47:464.

### Materials and Methods

The study was conducted at the four institutions indicated in Table 6. For reference control subjects, serum or plasma were collected from 109 healthy individuals, and the data were used to determine the 95^{th} percentile of a control reference population for the ACB Test.

Two hundred fifty six patients were recruited for the study. All patients arrived at the emergency rooms of participating institutions, and experienced clinical signs and symptoms of acute coronary syndrome within the prior three hours, as determined by medical record review. All enrolled patients had blood collected within 1 hour of presentation, and at least one other specimen collected between 6 and 24 hours later. The ACB Test and a cTnI assay were performed for each presentation sample; all enrolled patients had a negative cTnI result for the early sample, as classified by the cutoffs listed in Table 6. Troponin I testing was also performed for all samples from the 6-24 hour timeframe.

**Table 6. Centers participating in the study and associated data.**

| Institution | cTnI method (cutpoint) | No. suspected ACS* patients | No. control subjects | CV of ACB Test, % | Instrument Utilized |
|---|---|---|---|---|---|
| Hennepin Cty. Med. Ctr, Minneapolis, MN | Vitros ECi (>0.8ng/L) | 63 | 27 | 6.0 | COBAS FARA |
| Hartford Hospital, Hartford, CT | Vitros Eci (>0.8 ng/L) | 84 | | 8.7 | COBAS FARA |
| Univ. TN, Knoxville, TN | AxSYM (>1.6 ng/L) | 65 | 32 | 7.1 | COBAS MIRA Plus |
| Univ. MD, Baltimore, MD | Dimension RxL (>1.5 ng/L) | 44 | 50 | 7.4 | COBAS MIRA Plus |

| | | | | | |
|---|---|---|---|---|---|
| *ACS=acute coronary syndrome | | | | | |

The design of the study required knowledge of the timeframe and nature of acute cardiac ischemia with as much confidence as possible. For this reason, 32 of the 256 patients enrolled were excluded from analysis because of uncertainties surrounding their clinical event. Of these 32 patients, 8 were excluded because MI may have occurred more than 3 hours before presentation, as indicated by increased values of other biochemical markers, including myoglobin and/or cTnT at the time of admission. Sixteen other patients were excluded because of uncertainty in that their cTnI results did not match other biochemical marker data in the 6-24 hour timeframe. In the remaining 8 of these 32 patients, ACB Test results were negative at presentation, but cTnI results were positive 12-24 hours later. Categorization was indeterminate because no specimen was available in the 6-12 hours timeframe to determine whether ischemia and MI had occurred by the time of presentation, in which case the negative ACB Test would be classified as falsely negative, or at a time after presentation, in which case the negative ACB Test result would be classified as a true negative. The study population comprised the remaining 224 patients included in data analysis.

Blood was collected in red-top or green-top Vacutainer Tubes, containing no anticoagulant or containing lithium heparin, respectively. Specimens were routinely centrifuged within 2 hours of collection for 10 min at 1000g, and serum or heparinized plasma was harvested. Specimens were stored at 2-8 °C for a maximum of 2 weeks; if a delay in testing was anticipated, samples were frozen at -20 °C or colder within 96 hours. Frozen samples were mixed thoroughly after thawing and recentrifuged before analysis. Specimens handled in this way showed no significant loss of recovery. Repeat freeze-thaw cycles were avoided.

The cTnI assays used at individual sites are listed in Table 6: the Abbott AxSYM cTnI system (Abbott Diagnostics Inc., Abbott Park, IL); the Dimension RxL cTnI system (Dade-Behring, Inc, Glasgow, DE); and the Vitros ECi cTnI (Ortho Clinical Diagnostics, Raritan, NJ). The typical imprecision (CV) of each troponin assay was less than 8% at the cutoffs listed in Table 6.

Patients were considered troponin positive if any sample collected during the 6-24 hour period exceeded the institutional cutoff listed in Table 6.

For the ACB test, serum or heparinized plasma (500 µL) was added to a centrifuge tube containing 0.45 g of CaCl₂. Without pre-mixing, the sample and CaCl₂ were centrifuged for 10 min. at 1000-1200g. After centrifugation, 300 µL of the resulting supernatant was transferred to a COBAS MIRA or FARA sample cup (Roche Diagnostics), taking care not to resuspend the CaCl₂. This pretreatment procedure was performed to remove chelators used as preservatives that might be present in samples from patients receiving intravenous medications.

For the ACB Test, all measurements were performed using either the COBAS MIRA or FARA instrument systems (Roche Diagnostics); Table 6 lists the instrument system used at each site. Maintenance and operation of instruments were performed in accordance with the manufacturer's specifications.

In the ACB Test method, 95 µL of a patient's sample and 5 µL of Co(II), in the form of cobalt chloride, were incubated in the instrument cuvette for 5 min. During incubation, the Co(II) (final concentration, 0.58 mmol/L) binds to the NH₂ terminus of unaltered albumin in the sample; albumin for which the NH₂ terminus is altered as a result of ischemic processes binds the added Co(II) to a far lesser extent (Bar-Or et al. (1999) Ann. Emerg. Med. 34:S56; Bar-Or et al. (2000) J. Emerg. Med. 19:311). After incubation, 25 µL of dithiothreitol is added to the mixture. Dithiothreitol (final concentration, 1.67 mmol/L) forms a colored complex with Co(II) that is not bound at the NH₂ terminus of albumin, and this complex can be measured spectrophotometrically at 500 nm. The ACB Test results were obtained from a calibration curve produced using five calibrators with assigned ACB Test values of 6-186 units/mL. The ACB Test was designed so that all samples are measured in duplicate, with the higher reading being the result of the assay.

The imprecision (CV) of the ACB Test was calculated from the duplicate results for each sample at each test site.

The categorization criteria of the ACB Test results were as follows. If the ACB Test was equal to or above the cutoff or decision point determined using the Receiver Operating Characteristics (ROC) curve analysis, then the result was positive; otherwise the test result was negative. True-positive results occurred when the ACB Test was positive and the cTnI result for the subsequent 6-24 hour sample(s) was also positive. A true-negative result occurred when the ACB Test was negative and the cTnI result was also negative for the next sample(s) collected within the subsequent 6 to 12 hours. A false-positive result occurred when the ACB Test was positive but the cTnI results for samples collected in the subsequent 6-24 hours were negative. A false-negative result occurred when the ACB Test was negative and the cTnI result within the subsequent 6-12 hours was positive.

For statistical analysis, ROC curve analysis and calculation of the area under the curve was done for the ACB Test in the 224 patients included in the study population according to the method of Hanley and McNeil (Hanley et al. (1982) Radiology 143:29). The optimum cutoff for the ACB Test was selected from the ROC analysis to minimize the number of false-positive and false-negative results in this study population. This optimum cutoff was used to dichotomously classify each patient as ACB Test positive or ACB Test negative. Diagnostic sensitivity, specificity, positive predictive value (PPV), and negative predictive value (NPV) were calculated to determine the ability of the dichotomous ACB Test value at presentation to predict a later positive or negative troponin value at 6-24 hours after presentation. The exact 95% confidence intervals (95% CIs) were calculated using binomial distribution statistics. Goodness of fit to a gaussian distribution for the ACB Test results for the control reference population was evaluated using the χ² method. The upper 95th percentile ACB Test value for apparently healthy individuals was calculated using parametric statistics. The Wilcoxon rank test was used to compare the ACB Test values between the cTnI-positive and cTnI-negative patients. P<0.05 was considered significant.

### Results

The numbers of suspected acute coronary syndrome and healthy control subjects enrolled at each site are shown in Table 6. Table 6 also shows that the CVs for the ACB Test at each site were similar (mean CV, 7.3%; range, 6.0 - 8.7%).

The ACB Test values for the control reference population were normally distributed (χ² = 0.693; P = 0.9946). Values for the control reference population (n = 109) were 25.7-84.5 units/mL (mean, 58.7 units/mL; median, 59.5 units/mL). The upper 95th percentile was 80.2 units/mL.

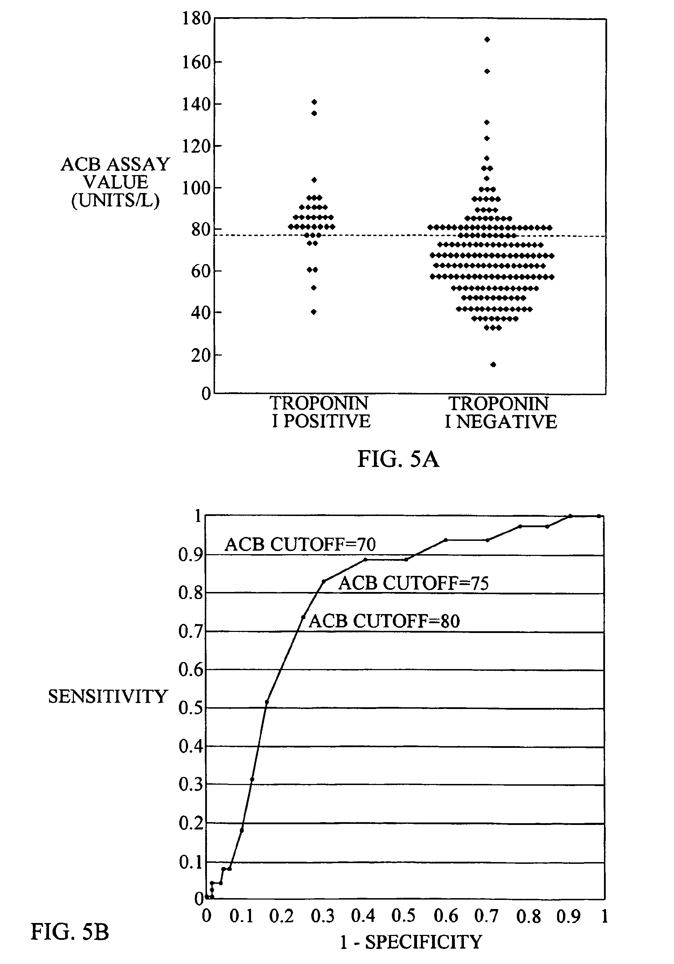

ACB Test results for the 224 acute coronary syndrome patients are shown in Fig. 5. Fig. 5A displays the distribution of the ACB Test results that were used to plot the ROC curve shown in Fig. 5B. Differences in the ACB Test results between the cTnI-positive and - negative patients (Fig. 5A) were highly significant (P<0.00001). The area under the ROC curve (Fig. 5B) was 0.78 (95% CI, 0.70-0.86). The optimum decision point for the ACB Test was determined to be 75 units/mL, and this decision limit was used in subsequent analyses.

ACB Test data for the 32 patients excluded from this study were not analyzed because of issues described above.

Table 7 displays truth tables for the ACB Test using a cutoff of ≥75 units/mL. The data shown yielded a sensitivity for the ACB Test of 83% (95% CI, 66-93%), a specificity of 69% (95% CI, 62-76%), a PPV of 33% (95% CI, 24-44%), and a NPV of 96% (95% CI, 91-98%).

**Table 7. Truth table for the ability of the ACB Test result at patient presentation to predict troponin-positive or -negative results at 6-24 hours after presentation.**

| | Troponin positive | Troponin negative | Total |
|---|---|---|---|
| ACB Test positive | 29 (TP*) | 58 (FP) | 87 (TP + FP) |
| ACB Test negative | 6 (FN) | 131 (TN) | 137 (FN + TN) |
| Total | 35 (FN + TP) | 189 (FP + TN) | 224 (TP+ TN + FP + FN) |

| | | | |
|---|---|---|---|
| *TP, true positive; FP, false positive; FN, false negative; TN, true negative | | | |

The ACB Test decision limit of 75 units/mL was lower than the 80.2 units/mL value representing the upper 95^{th} percentile of the control reference population included in the study. ("Units" or "U" refers to arbitrary units based on spectrophotometric absorption in the instrument.) Although the difference between the cTnI positive and cTnI negative groups was highly significant, the ACB Test values between the groups overlapped (Fig. 5A). Use of an ACB Test cutoff of 75 units/mL was a balance between maximizing sensitivity and the tradeoff of increasing false-positive results. This lowered diagnostic specificity (69%) and the PPV (33%). Overlap between high-risk (disease) and control reference (non-disease) populations was not ideal, but such overlap is also seen with numerous other useful diagnostic laboratory tests.

In this study, diagnostic performance was derived from the same study population that was used to determine the optimum ACB Test decision limit (cutoff), and for calculating diagnostic sensitivity, specificity, PPV and NPV. Therefore, diagnostic performance values may be refined as additional studies are conducted.

### Example 2 - Use of the ACB Test and Troponin Assay in Diagnosis of AMI

Using the data generated from patients described in Example 1 and continuing clinical trial data from additional patients recruited using the same protocol, the utility of the ACB Test as an aid to the diagnosis of AMI in patients presenting with chest pain or other symptoms of suspected cardiac ischemia was evaluated. Data from a total of 318 patients were analyzed. Each patient presented to a hospital emergency department with chest pain present or other symptoms within the previous three hours, although the time of onset of chest pain could have been earlier.

Both fresh and stored samples were used. All chest pain patients had multiple blood draws for cardiac markers (i.e., troponin) consistent with the hospital's standard practice.

Results from all blood draws were partitioned into three time periods:
1. First blood draw at presentation (denoted as 0 hours);
2. All blood draws greater than 0 hours and less than or equal to 6 hours, only if there was a blood draw during that time period; and
3. All blood draws greater than 6 hours and less than or equal to 12 hours, only if there was a blood draw during that time period.

The data were analyzed for three cases:
1. ACB Test alone;
2. Troponin I alone; and
3. Combination of Troponin I and the ACB Test (denoted as ACB & TnI in the following tables).

A positive troponin was defined as either TnI positive two or more sequential times during the draw period, or a single troponin ≥2 times the institutional cutoff for AMI.

The institutional diagnostic cutoffs for TnI for AMI for the four clinical trial sites are set forth in Table 6, *supra.*

A positive ACB Test was defined as a positive ACB Test at any time in any replicate during the draw period. The ACB Test cutoff used for the analyses was >80.00 U/mL.

Note that for both the ACB and TnI tests the definitions of a positive result are cumulative. Any positive result is carried forward from a previous result to all subsequent draws. For example, if the first draw was positive, but a 4 hour draw and a 10 hour draw were negative, then the >0 to 6 hour draw and the >6 to 12 hour draw would count as positive also.

Note also that a single TnI value was positive if either of the following was true: 1) the TnI value was at least twice the institutional cutoff, or 2) the TnI value was above the cutoff and had at least one previous draw also above the cutoff. For example, if TnI was greater than the institutional cutoff for the first and second draws but not greater than or equal to twice the institutional cutoff for the first draw, then it was considered negative for the first draw, and positive for the second draw. Although only a single cutoff point was considered for the analysis described herein, additional analyses were performed to investigate other cutoffs, such as the new ACC/ESC guidelines of using the 99^{th} percentile of upper limit of normal as the cutoff. The results were substantially the same as described below.

The data was analyzed for the performance of biochemical markers in predicting diagnosis of AMI as determined by the institutional discharge diagnosis. If the institutional discharge diagnosis was not reported, the ACC/ESC guidelines were used to determine a diagnosis of AMI.

The data for the first or presentation draw only for all patients are presented below as Tables 8-10 showing sensitivity, specificity, negative predictive value (NPV) and positive predictive value (PPV) for ACB only, Troponin-I only, and ACB and Troponin-I combined. 95% confidence intervals are shown.

The algorithm used to analyze the ACB and Troponin-I data combined is as follows:
• Diagnosis is positive for AMI if *either* ACB or troponin are positive;
• Diagnosis is negative for AMI if *both* ACB and troponin are negative.

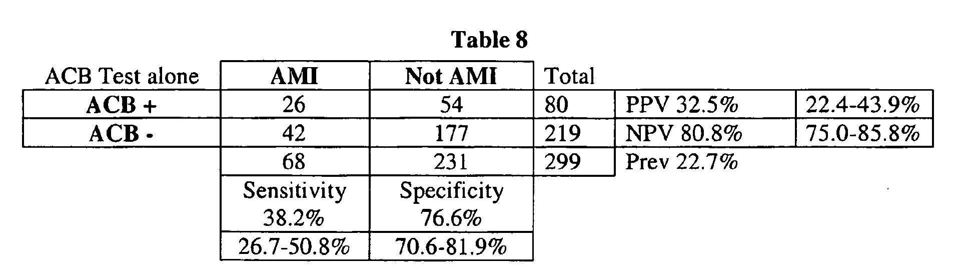

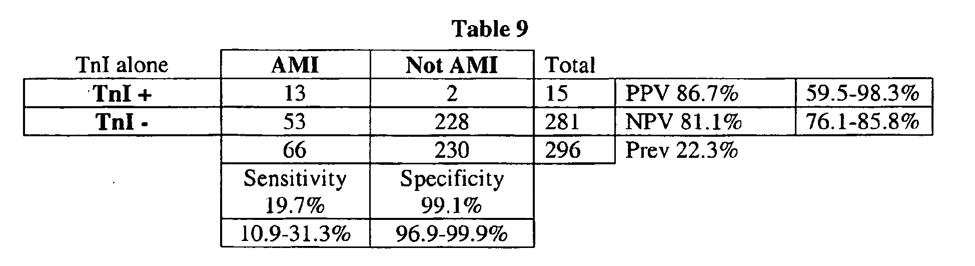

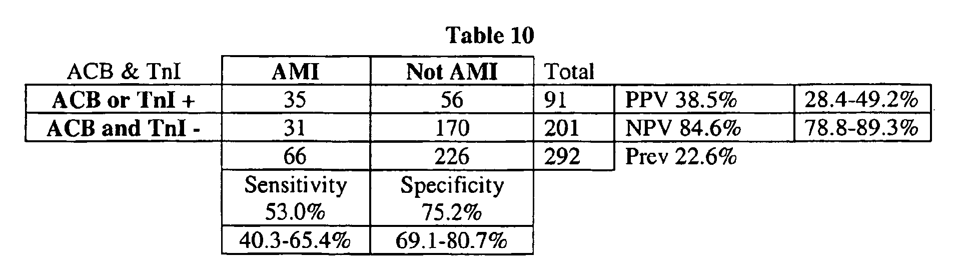

The graph in Figure 3 is a plot of sensitivity of ACB Test alone, Troponin-I alone, and the combination of the ACB Test and Troponin-I for each of the three draw periods of initial draw, blood draw from zero to six hours, and blood draws from six to twelve hours.

These data clearly show that the ACB Test alone has higher sensitivity for diagnosis of AMI than Troponin I alone, at least in the earlier blood draw times. Furthermore, the combination of the ACB Test and Troponin-I has higher sensitivity than either Troponin I or the ACB Test alone.

## Claims

1. A method for diagnosis of ischemia or myocardial infarction occurring in a patient's tissue comprising the steps of:
(a) conducting on a patient blood, serum or plasma sample that has been in contact with ischemic tissue at least one *in vitro* assay for ischemia modified albumin and at least one *in vitro* assay for a cardiac necrosis marker; and
(b) combining the results of the assays of step (a) using an algorithm to provide a diagnosis of ischemia or myocardial infarction, said algorithm comprising:
diagnosing ischemia with either early infarct of less than 4 - 6 hours or stable angina, if the modified albumin result is positive and the necrosis marker result is negative; and
diagnosing myocardial infarction if the ischemia modified albumin result is positive or negative and at least one of the necrosis marker results is positive.

2. The method of claim 1 wherein the assay for necrosis is selected from the group consisting of assays for troponin, CK-MB and myoglobin.

3. The method of claim 1 wherein the myocardial infarction of step (b) comprises an infarct of at least 6 hours, if the modified albumin result is negative and the necrosis marker test is positive.

4. The method of claim 1, wherein step (b) further comprises: making a negative diagnosis of ischemia or acute myocardial infarction, where the modified albumin result and all the necrotic marker results are negative.

5. The method according to any one of claims 1 to 4.wherein the assay is an albumin cobalt binding (ACB) test.

6. The method according to any one of claims 1 to 6 wherein the assay is a immunoassay for ischemia modified albumin.

## Patentansprüche

1. Verfahren zur Diagnose von Ischämie oder Herzinfarkt, die oder der im Gewebe eines Patienten auftritt, bei dem man
(a) an einer Blut-, Serum- oder Plasmaprobe des Patienten, die mit ischämischem Gewebe Kontakt hatte, mindestens einen in vitro Test für Ischämie-modifiziertes Albumin und mindestens einen in vitro Test für einen Herznekrose-Marker durchführt, und
(b) man die Ergebnisse der Tests von Schritt (a) unter Verwendung eines Algorithmus kombiniert, um eine Diagnose für Ischämie oder Herzinfarkt zu erhalten, wobei der Algorithmus
die Diagnose von Ischämie entweder mit einem frühen Infarkt von weniger als 4-6 Stunden oder stabiler Angina umfasst, wenn das Ergebnis für modifiziertes Albumin positiv und das Ergebnis für Nekrosemarker negativ ist, und
die Diagnose von Herzinfarkt umfasst, wenn das Ergebnis für Ischämie-modifiziertes Albumin positiv oder negativ und mindestens eines der Ergebnisse für Nekrosemarker positiv ist.

2. Verfahren nach Anspruch 1, bei dem der Nekrosetest aus der Gruppe ausgewählt ist, die aus Tests für Troponin, CK-MB und Myoglobin besteht.

3. Verfahren nach Anspruch 1, bei dem der Herzinfarkt in Schritt (b) einen Infarkt von mindestens 6 Stunden umfasst, wenn das Ergebnis für modifiziertes Albumin negativ und der Test für Nekrosemarker positiv ist.

4. Verfahren nach Anspruch 1, bei dem man in Schritt (b) weiterhin eine negative Diagnose für Ischämie oder akuten Herzinfarkt stellt, wenn das Ergebnis für modifiziertes Albumin und alle Ergebnisse für Nekrosemarker negativ sind.

5. Verfahren nach einem der Ansprüche 1 bis 4, bei dem der Test ein Albumin-Kobaltbindungstest (ACB-Test) ist.

6. Verfahren nach einem der Ansprüche 1 bis 5, bei dem der Test ein Immuntest für Ischämie modifiziertes Albumin ist.

## Revendications

1. Procédé pour le diagnostic de l'ischémie ou de l'infarctus du myocarde survenant dans un tissu de patient, comprenant les étapes de :
(a) conduire sur un échantillon de sang, de sérum ou de plasma du patient, qui a été en contact avec le tissu ischémique au moins un dosage *in vitro* d'albumine modifiée par l'ischémie et d'au moins un dosage *in vitro* d'un marqueur de nécrose cardiaque et
(b) combiner les résultats des dosages de l'étape (a) en utilisant un algorithme pour fournir un diagnostic de l'ischémie ou de l'infarctus du myocarde, ledit algorithme comprenant :
le diagnostic d'une ischémie avec un infarctus précoce de moins de 4 - 6 heures ou une angine de poitrine stable, si le résultat de l'albumine modifiée est positif et le résultat du marqueur de nécrose négatif ; et
le diagnostic de l'infarctus du myocarde si le résultat de l'albumine modifiée par l'ischémie est positif ou négatif et au moins un des résultats du marqueur de nécrose est positif.

2. Procédé de la revendication 1, dans lequel le dosage de la nécrose est choisi parmi le groupe consistant en les dosages de la troponine, de la CK-MB et de la myoglobine.

3. Procédé de la revendication 1, dans lequel l'infarctus du myocarde de l'étape (b) comprend un infarctus d'au moins 6 heures, si le résultat de l'albumine modifiée est négatif et le test du marqueur de nécrose est positif.

4. Procédé de la revendication 1, dans lequel l'étape (b) comprend en outre : l'établissement d'un diagnostic négatif de l'ischémie ou de l'infarctus aigu du myocarde, lorsque le résultat de l'albumine modifiée et tous les résultats du marqueur nécrotique sont négatifs.

5. Procédé selon l'une quelconque des revendications 1 à 4 dans lequel le dosage est un test de fixation du cobalt sur l'albumine (test ACB).

6. Procédé selon l'une quelconque des revendications 1 à 5, dans lequel le dosage est un immuno-essai de l'albumine modifiée par une ischémie.
